# EUROPEAN PATENT APPLICATION

(11) **EP 1 186 668 A1**
(43) Date of publication of application: **13.03.2002**
(21) Application number: 00203117.7
(22) Date of filing: 08.09.2000
(51) Int. Cl.: C12P 35/02, C12P 35/04, C12P 37/04, C12P 37/06, C12P 39/00

(54) **An enzymatic process for preparing Beta-lactam compounds**

(71) Applicant: DSM N.V., 6160 MA Geleen (NL)
(72) Inventor: van der Does, Thomas, 2613 ZA Delft (NL)

(57) **Abstract**

An enzymatic process for the preparation of a semi-synthetic β-lactam antibiotic compound by reacting an N-substituted β-lactam with two enzymes, namely a penicillin acylase and an α-amino acid ester hydrolase or microorganism expressing these enzymes, with a precursor for the side chain of the β-lactam antibiotic compound has been described.

## Description

### Field of the invention

The present invention relates to an enzymatic process for the preparation of a semi-synthetic β-lactam antibiotic compound by reacting an N-substituted β-lactam with a precursor for the side chain of the β-lactam antibiotic compound.

### Background of the invention

β-Lactam antibiotic compounds constitute the most important group of antibiotic compounds, with a long history of clinical use. Among this group, the prominent β-lactam antibiotic compounds are the penicillins and cephalosporins. In general, β-lactam antibiotic compounds, or, in short, β-lactam antibiotics consist of a nucleus, the so-called amino β-lactam nucleus, which is linked through its primary amino group to the so-called side chain *via* a linear amide bond.

Presently, most of the β-lactam antibiotics used are prepared by semi-synthetic methods. These semi-synthetic β-lactam antibiotics are obtained by modifying an N-substituted β-lactam product by one or more chemical and/or enzymatic reactions. In this text, an N-substituted β-lactam is defined as a compound according to formula 1. Typically, one of the reactions involved in the modification of the N-substituted β-lactam is acylation of the amino β-lactam nucleus, which has been obtained from the N-substituted β-lactam.

Conventionally, β-lactam antibiotics have been prepared in chemical procedures. However, such chemical methods have a number of grave disadvantages. They comprise many complex reactions, wherein by-products are formed which give rise to effluent and purification problems. Also, because many steps have to be performed in order to obtain the final antibiotic product, the overall yield is quite low.

Recently, there has been a lot of interest for so-called enzymatic semi-synthetic routes to β-lactam antibiotics. These routes involve enzymatically-catalyzed processes and lack many of the disadvantages of the conventional synthetic methods for preparing β-lactam antibiotics. The enzymatically-catalyzed reactions are highly selective, thus the production of many by-products, and the effluent and purification problems, which result therefrom, are reduced or avoided. Furthermore, enzymatic processes can be performed in aqueous environment.

The semi-synthetic routes mostly start from fermentation products such as isopenicillin N, penicillin G, penicillin V and cephalosporin C, which products are enzymatically converted to an amino β-lactam nucleus, for instance in a manner as has been disclosed in *Bioprocess. Technol.,* **16**, 67-88 (1993) by K. Matsumoto, in *Process Biochemistry,* 146-154 (1989) by J.G. Shewale & H. Sivaraman, in Biotechnology of Industrial Antibiotics (Ed. E.J. Vandamme) Marcel Dekker, New York (1984) by T.A. Savidge, or in *Process Biochemistry International,* June, 97-103 (1990) by J.G. Shewale et al. The obtained amino β-lactam nucleus is subsequently converted to the desired β-lactam antibiotic by coupling the nucleus to a suitable side chain, as has been described in *inter alia* German patent application No. DE 2163792, Dutch patent No. NL 158847 B, German patent No. DE 2621618 C, European patent No. EP 339751 B and International patent applications No. WO 9201061 and WO 9312250. Enzymatic acylation of an amino β-lactam nucleus with a side chain precursor which is a carbonyl-activated derivative *(i.e.* amide or alkyl ester) of a side chain acid has also been described in *Biochem. J.* (1969) 747-756 by M. Cole and in Ann. N.Y. Acad. Sci. 99-105 (1996) by V. Kasche *et al.* By making different combinations of side chains and amino β-lactam nuclei, a variety of penicillin and cephalosporin antibiotics may be obtained. For example, a D-(-)-phenylglycine side chain may be attached to a 6-aminopenicillanic acid (6-APA) nucleus, in a reaction with a suitable derivative of said D-(-)-phenylglycine, to yield ampicillin, or to a 7-aminodesacetoxycephalosporanic acid (7-ADCA) nucleus to yield cephalexin.

A disadvantage of the known methods is that the coupling reaction starts from an amino β-lactam nucleus, which has been isolated prior to the coupling reaction. During the isolation of the amino β-lactam nucleus, which is usually performed by crystallization, up to about 10% of the theoretical yield is lost. Due to the amphoteric nature of the β-lactam nucleus, it dissolves readily in an aqueous environment at any pH value. As a result a great part of the production of the β-lactam nucleus is lost in the mother-liquor resulting from the crystallization.

In the International patent application WO 98/48038, a process for preparing a β-lactam antibiotic is described wherein the antibiotic is prepared from a mixture in which a N-substituted β-lactam a dicarboxylate acylase and a penicillin acylase and a desired side chain precursor. The process includes the in-situ preparation of a β-lactam nucleus which is formed when the N-subtituted β-lactam is liberated enzymatically from its side chain. In WO 98/48038, however, the N-substituted β-lactam starting material is limited to a β-lactam compounds containing a succinyl, glutaryl or an adipyl or analogous thereof as the N-substituent.

Furthermore, R. Okachi and T. Nara in *Agr. Biol. Chem*., **37** (12), 2797-2804 (1973) have described that an enzyme derived from *P. melanogenum* is quite useful for practical preparation of ampicillin by enzymatic acylation, because it is possible then to use a reaction mixture containing 6-aminopenicillanic acid and phenylacetic acid resulting from the cleavage of penicillin G by *K. citrophila* acylase. However, the publication does not disclose other enzymes suitable for combination in such a process.

The object of the invention is to provide an enzymatic method for preparing a β-lactam antibiotic, which method is preferably also efficient and in which method a large variety of N-phenylacetyl β-lactams or N-phenoxyacetyl β-lactams may be used as a starting material.

Surprisingly, it has been found that an enzymatic acylation of the *in situ* prepared amino β-lactam nucleus with a precursor for the side chain of the β-lactam antibiotic compound for the production of the corresponding β-lactam antibiotic can advantageously be carried out with two enzymes, one of which is a penicillin acylase and the other one of which is an α-amino acid ester hydrolase, or microorganisms expressing these enzymes.

### Description of the invention

The present invention describes a process for the production of a β-lactam antibiotic compound, wherein an N-substituted β-lactam, having the general formula (I): 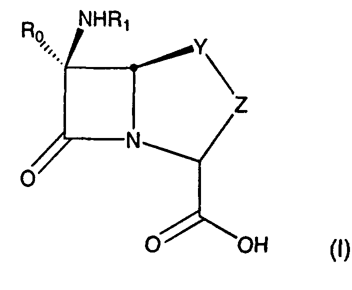 wherein
R₀ is hydrogen or C₁₋₃ alkoxy;
R₁ is a phenylacetamido or phenoxyacetamido;
Y is CH₂ or a substituted CH₂, oxygen, sulfur, or an oxidized form of sulfur; and
Z is 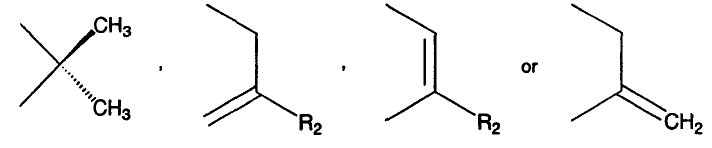 wherein R₂ is hydrogen, hydroxy, halogen, C₁₋₃ alkoxy, C₁₋₅ alkyl, C₅₋₈ cycloalkyl optionally containing one or more heteroatoms,
   aryl or heteroaryl, or benzyl,
   is contacted with at least one penicillin acylase or microorganisms expressing such an enzyme and reacted with a precursor for the side chain of the β-lactam antibiotic compound in water or in a mixture of water and one or more organic solvents in the presence of an α-amino acid ester hydrolase or microorganisms expressing such an enzyme.

When R₂ is a C1-5 alkyl group, this alkyl group may be saturated or unsaturated, branched or straight, and it is optionally substituted or optionally it contains one or more heteroatoms. Preferably, the alkyl group is a methyl. The C₅₋ ₈cycloalkyl is optionally substituted, it optionally contains one or more hetero atoms. The aryl group is optionally substituted. The benzyl group may also be substituted or unsubstituted.

The formula (I) is intended to encompass the *N*-substituted β-lactams, which are based on any β-lactam nucleus disclosed in "Cephalosporins and Penicillins, Chemistry and Biology", Ed. E.H. Flynn, Academic Press, 1972, pages 151-166, and "The Organic Chemistry of β-Lactams", Ed. G.I. Georg, VCH, 1992, pages 89-96, which are incorporated herein by reference. A side chain refers to a substituent present on the amino group of the amino β-lactam nucleus, which combination together constitutes the β-lactam antibiotic end product.

Preferred *N*-substituted β-lactams are *N*-substituted 6-aminopenicillanic acid, 7-aminocephalosporanic acid, 7-amino-3-chloro-3-(desacetoxymethyl)- cephalosporanic acid, 7-amino-3'-desacetoxycephalosporanic acid, 7-amino-3'-desacetylcephalosporanic acid, 7-amino-3'-[(1-(2-dimethylamino)ethyl)-1 *H*-tetrazol-5-ylthio]-3'-desacetoxycephalosporanic acid, 7-amino-3'-methoxy-3'-desacetoxycephalosporanic acid, 7-amino-3-methoxy-3-(desacetoxymethyl)- cephalosporanic acid, 7-amino-3'-(1-methylpyrrolidin-1-yl)-3-(desacetoxy- methyl)cephalosporanic acid, 7-amino-3'-(1 -methyl-1*H*-tetrazol-5-ylthio)-3'-desacetoxycephalosporanic acid, 7-amino-3'-(5-methyl-1,3,4-thiadiazol-2-ylthio)-3'-desacetoxycephalosporanic acid, 7-amino-3-[(*Z*)-1-propenyl]-3-(desacetoxymethyl)cephalosporanic acid, 7-amino-3'-(1,2,3-triazol-4-ylthio)-3'-desacetoxycephalosporanic acid, 7-amino-3-vinyl-3-(desacetoxymethyl)-cephalosporanic acid and (6*R*,7*R*)-3-chloro-7-amino-8-oxo-1-azabicyclo-[4.2.0]oct-2-ene-2-carboxylic acid, or a salt thereof.

Examples of the *N*-substituted β-lactams which are usually prepared by fermentation are penicillin G, 7-[(phenylacetyl)amino]-3-(desacetoxymethyl)cephalosporanic acid and penicillin V. A great advantage of the present invention resides therein that it is now possible to prepare β-lactam antibiotics enzymatically, starting from such fermentation products, without the isolation of an amino β-lactam nucleus intermediate, which isolation causes a loss of product.

Suitable salts of the *N*-substituted β-lactam include any non-toxic salt, such as an alkali metal salt (e.g. sodium or potassium), an alkali earth metal salt (e.g. calcium or magnesium), an ammonium salt, or an organic base salt (e.g. trimethylamine, triethylamine, pyridine, picoline, dicyclohexylamine or *N,N*-dibenzyl diethylene diamine).

A suitable penicillin acylase, with which the *N*-substituted β-lactam is contacted in a method according to the invention, is an enzyme that may be classified as E. 3.5.1.11 and which may be isolated from various naturally occurring microorganisms, such as fungi and bacteria.

Microorganisms that have been found to produce penicillin acylase are, for example, *Acetobacter, Aeromonas, Alcaligenes, Aphanocladium,* *Bacillus* sp., *Cephalosporium, Escherichia, Flavobacterium, Kluyvera, Mycoplana, Protaminobacter, Pseudomonas* or *Xanthomonas* species. Enzymes derived from *Acetobacter pasteurianus, Alcaligenes faecalis, Bacillus megaterium* and *Escherichia coli* have particularly proven to be succesful in a method according to the invention. In the literature, penicillin acylases have also been referred to as penicillin amidases.

α-Amino acid ester hydrolase are classified as E 3.1.1.43. Microorganisms that have been found to produce α-amino acid ester hydrolase are obtained from for example an *Acetobacter, Pseudomonas* or a *Xanthomonas* species. The enzymes derived from *Acetobacter pasteurianus, Acetobacter turbidans, Pseudomonas aeruginosa, Pseudomonas melanogenum* or *Xanthomonas citri* have been proven to be particularly successful and more particularly the enzymes from the group consisting of *Acetobacter pasteurianus* ATCC 6033, *Pseudomonas melanogenum* KY 3987 or KY 8540 and *Xanthomonas citri* K24. α-amino acid ester hydrolase has been described, for example, in *Agr. Biol. Chem.,* **37** (12), 2797-2804 (1973) by R. Okachi and T. Nara, in *Biochem. J.,* **137**, 497-503 (1974) by T. Takeshi, Y. Yakahashi, Y. Yamazaki and K. Kato, in *Agr. Biol. Chem.,* **44** (4), 821-825 (1980) by K. Kato, K. Kawahara and S. Igarasi, in *Agr. Biol. Chem.*, **44** (5), 1069-1094 (1980) by K. Kato, K. Kawahara, T. Takahashi and A. Kakinuma, and in *Agr. Biol. Chem.,* **47** (11), 2813-2509 (1983) by M. Kawamori, Y. Hashimoto, R. Katsumata, R. Okachi and K. Takayama.

The penicillin acylase and the α-amino acid ester hydrolase may for example be used as free enzymes, but may also be used in any suitable immobilized form, for instance as has been described in European patent EP 222462 B and International patent application WO 97/04086. It is possible to perform a method according to the invention wherein both enzymes are immobilized on one carrier or wherein the enzymes are immobilized on different carriers. Preferably, the penicillin acylase is used in an immobilized form. In addition, it is possible to use functional equivalents of one or both of the enzymes, wherein for instance properties of the enzymes, such as pH dependence, thermostability or specific activity may be affected by chemical modification or cross-linking, without significant consequences for the activity, in kind, not in amount, of the enzymes in a method according to the invention. Also, functional equivalents such as mutants or other derivatives obtained by classic means or *via* recombinant DNA methodology, biologically active parts or hybrids of the enzymes may be used. In some cases, modification, chemical or otherwise, may be beneficial in a method according to the invention, as is part of the standard knowledge of the person skilled in the art.

The precursor for a side chain of the β-lactam antibiotic to be prepared in a method according to the invention may be any compound that is recognized by the above defined α-amino acid ester hydrolase and leads to a product of the class of β-lactam antibiotics. Preferably, the precursor is chosen from the group of D-(-)-phenylglycine, D-(-)-4-hydroxyphenylglycine, D-(-)-2,5-dihydrophenylglycine, 2-thienylacetic acid, 2-(2-amino-4-thiazolyl)-2-methoxyiminoacetic acid, a-(4-pyridylthio)acetic acid, 3-thiophenemalonic acid, or 2-cyanoacetic acid, and derivatives thereof, as these side chain precursors, after reaction with an amino β-lactam nucleus, lead to β-lactam antibiotics having the most advantageous activity profile. Suitable derivatives of these side chain precursors are for example esters and amides. Preferably, the C₁₋₃-alkyl esters of D-(-)-phenylglycine, D-(-)-4-hydroxyphenylglycine, D-(-)-2,5-dihydrophenylglycine, 2-thienylacetic acid, 2-(2-amino-4-thiazolyl)-2-methoxyiminoacetic acid, a-(4-pyridyithio)acetic acid, 3-thiophenemalonic acid, or 2-cyanoacetic acid are used. The most preferred side chain derivatives are the methyl esters. In a method according to the invention, the penicillin acylase or microorganisms expressing this enzyme, the precursor for the side chain of the β-lactam antibiotic and the α-amino acid ester hydrolase or microorganisms expressing that enzyme may be added to the *N*-substituted β-lactam starting material together or seperately. Preferably, the enzymes are added seperately from the side chain precursor to the *N-*substituted β-lactam.

In an embodiment, the N-substituted β-lactam is contacted with the penicillin acylase in a reaction vessel, and subsequently the resulting reaction mixture is added to a second vessel in which the α-amino acid ester hydrolase and the precursor for the side chain are present. When the penicillin acylase is used in an immobilized form, it may optionally be removed, for example by filtration, before adding the rest of the reaction mixture to the second vessel. In this way it is possible to carry out both enzymatic reactions at their optimum pH.

In a preferred embodiment of the invention, the preparation of a β-lactam antibiotic is performed by introducing the precursor for the side chain in the reaction catalyzed by an α-amino acid ester hydrolase with an amino β-lactam nucleus produced *in situ* by the reaction of a penicillin acylase with the *N*-substituted β-lactam starting material. This embodiment is an example of a one-pot process. By "one-pot process" any process is meant wherein the complete process is carried out in one reactor vessel.

In a preferred embodiment of the invention, a process is carried out as an one-pot process without isolation and/or purification of any intermediates that may at one time or another be present in the reaction mixture. This way, no product is lost in an isolation or purification process. In addition, the equilibrium of the reaction wherein the amino β-lactam nucleus is produced from the N-substituted β-lactam compound, will shift to the side of amino β-lactam nucleus-production, because the nucleus reacts with the side chain precursor. This results in a higher efficiency in the formation of the nucleus. The advantages of one-pot processes will be evident to the skilled person.

The conditions applied in a method according to the invention depend on various parameters, in particular the type of reagents, the concentration of reagents, reaction time, titrant, temperature, pH, enzyme concentration, and enzyme morphology. Given a specific *N*-substituted β-lactam that is to be converted to a given β-lactam antibiotic using a given α-amino acid ester hydrolase and a penicillin acylase, the person skilled in the art will be able to suitably choose the optimum reaction conditions.

It has, however, been found that the reaction temperature in a method according to the invention preferably lies between 0°C and 40°C, and more preferably between 5°C and 25°C. The pH in the preparation of the amino β-lactam nucleus according to the invention lies preferably between 4 and 9, more preferably between 5 and 8.5 and for the preparation of the antibiotic β-lactam compound thereof preferably between 4 and 9, more preferably between 5 and 7. In this regard, it is to be noted that is highly preferable to perform a method according to the invention in aqueous environment, because in this way the use of organic solvents, which would lead to effluent problems, is circumvented. Moreover, both the α-amino acid ester hydrolase and the penicilline acylase enzymes have proven to catalyze the conversion reaction most efficiently in an aqueous environment.

The pH-value during the reaction of the N-substituted β-lactam with the precursor for the side chain of the β-lactam antibiotic compound is preferably maintained constant. This is achieved for example by the addition of an inorganic base selected from the group consisting of sodium hydroxide, potassium hydroxide and ammonium hydroxide or an organic base such as the precursor for the side chain of the β-lactam antibiotic compound in the ester form. The base concentration may vary depending on the scale of the reaction and the solubility of the base.

Examples of β-lactam antibiotics, which can be produced by the process of this invention, are penicillins, preferably amoxicillin, ampicillin, epicillin and cephalosporins, preferably cefaclor, cefadroxil, cefatrizine, cefoperazon, cefprozil, cefetamet, cefotaxim, cephalexin, cephaloglycin, cephradine and carbacef, preferably loracarbef.

Surprisingly, no significant enzyme inhibition effect occurs in a method according to the invention. Up to now, it has been believed that transacylation using one or two enzymes in the preparation of β-lactam antibiotics is not possible due to an enzyme inhibition effect. It was expected that in the transacylation reaction, in which phenylacetic acid or phenoxyacetic acid are formed, these acids would act as inhibitors for certain enzymes as has been reported in *Applied and Environment Microbiology,* February, 307-312 (1984) by U. Schömer et al. and in *Biochim. Biophys. Acta*, **616**, 283-289 (1980) by A.L. Margolin et al.

Of course the invention also encompasses β-lactam antibiotic compounds obtainable by the methods disclosed hereinabove. The invention will now be described with reference to the following example, which is not to be constructed as being limiting on the invention, and are provided purely for illustrative purposes.

### Definitions

### α-Amino acid ester hvdrolase

Definition of unit ASU (Amoxicillin Synthesis Unit): 1 ASU corresponds to the amount of enzyme that produces 1 g amoxicillin trihydrate in 1 hour in a mixture of 6.26 g D-(-)-4-hydroxy- phenylglycine methyl ester (HPGM) and 6.49 g 6-aminopenicillanic acid (6-APA) in 100 ml water at 20°C.

### Penicillin acylase

Definition of unit PAU (Pen Acylase Unit): 1 PAU corresponds to the amount of enzyme that hydrolyses per minute 1 micromole penicillin G under standard conditions (100 g/l Penicillin G, potassium salt, 0.05 M potassium phosphate buffer, pH value 8.0 and 28°C).

### Example

### Enzymatic synthesis of amoxicillin starting from penicillin G

Penicillin acylase from *Escherichia coli* ATCC 11105 (0.08 ml, containing 52 PAU) was added to a solution of potassium salt of penicillin G (0.12 g) in water (8 ml) at room temperature. The pH was kept at 6.5 by the addition of solid D-(-)-4-hydroxy- phenylglycine methyl ester (HPGM). After 30 minutes, a cell free extract of *Acetobacter pasteurianus* ATCC 6033 (2 ml, containing 0.042 ASU) was added. Again the pH was kept at 6.5 by the addition of HPGM. After 4 hours, the addition of HPGM was stopped. After 24 hours, 0.16 mmol amoxicillin (50% yield based on penicillin G potassium salt) was formed as determined by HPLC.

## Claims

1. A process for the production of a β-lactam antibiotic compound, wherein an N-substituted β-lactam, having the general formula (I): 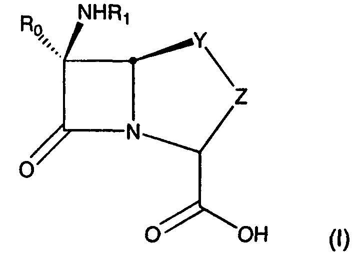 wherein
R₀ is hydrogen or C₁₋₃ alkoxy;
R₁ is a phenylacetamido or phenoxyacetamido
Y is CH₂ or a substituted CH₂, oxygen, sulfur, or an oxidized form of sulfur; and
Z is 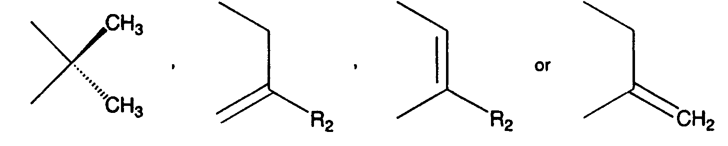
wherein R₂ is hydrogen, hydroxy, halogen, C₁₋₃ alkoxy, C₁₋₅ alkyl, C₅₋₈ cycloalkyl optionally containing one or more heteroatoms,aryl or heteroaryl, or benzyl,
is contacted with at least one penicillin acylase or microorganisms expressing such an enzyme and reacted with a precursor for the side chain of the β-lactam antibiotic compound in water or in a mixture of water and one or more organic solvents in the presence of an α-amino acid ester hydrolase or microorganisms expressing such an enzyme, with the proviso that a process for the production of ampicillin from penicillin G with α-aminophenylacetic acid methyl ester in the presence of penicillin acylase of *K*. *citrophila* and α-amino acid ester hydrolase of *Pseudomonas melanogenum* is excluded.

2. A process according to claim 1, which is performed as a one-pot process.

3. A process according to claim 1 or 2, wherein the penicillin acylase is immoblized.

4. A process according to anyone of the claims 1-3, wherein the penicillin acylase has been obtained from an *Acetobacter, Aeromonas, Alcaligenes, Aphanocladium, Bacillus* sp., *Cephalosporium, Escherichia, Flavobacterium, Kluyvera, Mycoplana, Protaminobacter, Pseudomonas* or a *Xanthomonas* species.

5. A process according to claim 1, wherein the α-amino acid ester hydrolase has been obtained from an *Acetobacter. Pseudomonas* or a *Xanthomonas* species.

6. A process according to anyone of the claims 1-8, wherein the *N*-substituted β-lactam is a *N*-substituted 6-aminopenicillanic acid, 7-aminocephalosporanic acid, 7-amino-3-chloro-3-(desacetoxymethyl)-cephalosporanic acid, 7-amino-3'-desacetoxycephalosporanic acid, 7-amino-3'-desacetylcephalosporanic acid, 7-amino-3'-[(1-(2-dimethylamino)ethyl)-1*H*-tetrazol-5-ylthio]-3'-desacetoxycephalosporanic acid, 7-amino-3'-methoxy-3'-desacetoxycephalosporanic acid, 7-amino-3-methoxy-3-(desacetoxymethyl)- cephalosporanic acid, 7-amino-3'-(1-methylpyrrolidin-1-yl)-3-(desacetoxy- methyl)cephalosporanic acid, 7-amino-3'-(1-methyl-1*H*-tetrazol-5-ylthio)-3'-desacetoxycephalosporanic acid, 7-amino-3'-(5-methyl-1,3,4-thiadiazol-2-ylthio)-3'-desacetoxycephalosporanic acid, 7-amino-3-[(*Z*)-1-propenyl]-3-(desacetoxymethyl)cephalosporanic acid, 7-amino-3'-(1,2,3-triazol-4-ylthio)-3'-desacetoxycephalosporanic acid, 7-amino-3-vinyl-3-(desacetoxymethyl)- cephalosporanic acid and (6*R*,7*R*)-3-chloro-7-amino-8-oxo-1-azabicyclo[4.2.0]oct-2-ene-2-carboxylic acid, or a salt thereof.

7. A process according to claim 6, wherein the *N*-substituted β-lactam is penicillin G, penicillin V, 7-[(phenylacetyl)amino]-3-(desacetoxymethyl)-cephalosporanic acid or 7-[(phenoxyacetyl)amino]-3-(desacetoxymethyl)-cephalosporanic acid.

8. A process according to anyone of the claims 1-7, wherein the precursor for the side chain of the β-lactam antibiotic is obtained from the group consisting of D-(-)-phenylglycine, D-(-)-4-hydroxyphenylglycine, D-(-)-2,5-dihydrophenylglycine, 2-thienylacetic acid, 2-(2-amino-4-thiazolyl)-2-methoxyiminoacetic acid, α-(4-pyridylthio)acetic acid, 3-thiophenemalonic acid and 2-cyanoacetic acid or an amide or ester of any of these compounds.

9. A process according to anyone of the claims 1-8, wherein the temperature during the reaction of the N-substituted β-lactam with a precursor for the side chain of the β-lactam antibiotic compound is maintained between 0ºC and 40ºC, preferably between 5ºC and 25ºC.

10. A process according to anyone of the claims 1-9, wherein the pH-value during the reaction of the N-substituted β-lactam with a precursor for the side chain of the β-lactam antibiotic compound is maintained between 5 and 8, preferably between 6 and 7 by the addition of an inorganic or organic base.

11. A process according to anyone of the claims 1-10 wherein the β-lactam antibiotic compounds are penicillins, preferably amoxicillin, ampicillin and epicillin.

12. A process according to anyone of the claims 1 -11 wherein the β-lactam antibiotic compounds are cephalosporins, preferably cefaclor, cefadroxil, cefatrizine, cefoperazon, cefprozil, cefetamet, cefotaxim, cephalexin, cephaloglycin, cephradine and loracarbef.

13. Use of an α-amino acid ester hydrolase *from Acetobacter pasteurianus* ATCC 6033, *Pseudomonas melanogenum* KY 3987 or *Pseudomonas melanogenum* KY 8540 and a penicillin acylase to convert an *N*-substituted β-lactam to a β-lactam antibiotic compound in a one-pot process.

14. Pharmaceutical composition comprising a β-lactam antibiotic compound prepared according to any one of the claims 1-13.
